(19) **Europäisches Patentamt**
**European Patent Office**
**Office européen des brevets**

(11) **EP 3 602 070 B1**

(12) **EUROPEAN PATENT SPECIFICATION**

(45) Date of publication and mention
of the grant of the patent:
**04.05.2022 Bulletin 2022/18**

(21) Application number: **18725634.2**

(22) Date of filing: **29.03.2018**

(51) International Patent Classification (IPC):
***G01N 33/68*** *(2006.01)*      ***G01N 33/86*** *(2006.01)*

(52) Cooperative Patent Classification (CPC):
**G01N 33/6893; G01N 33/86;** G01N 2800/222

(86) International application number:
**PCT/IB2018/052179**

(87) International publication number:
**WO 2018/178920 (04.10.2018 Gazette 2018/40)**

(54) **A RAPID, ON-DEMAND HEPARIN-INDUCED THROMBOCYTOPENIA FUNCTIONAL ASSAY**

SCHNELLER ON-DEMAND-FUNKTIONSTEST FÜR HEPARININDUZIERTE
THROMBOZYTOPENIE

DOSAGE FONCTIONNEL RAPIDE ET À LA DEMANDE DE LA THROMBOCYTOPÉNIE INDUITE
PAR L'HÉPARINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.03.2017 US 201762478105 P**

(43) Date of publication of application:
**05.02.2020 Bulletin 2020/06**

(73) Proprietor: **Emosis**
**67400 Illkirch-Graffenstaden (FR)**

(72) Inventors:
• **ALKHALFIOUI, Fatima**
**67400 Illkirch Graffenstaden (FR)**
• **GEORGE, Florian**
**67540 Ostwald (FR)**
• **TOMER, Aaron**
**5253137 Ramat Gan (IL)**

• **ALLEMAND, Frederic**
**67870 Griesheim Pres Molsheim (FR)**

(74) Representative: **Beck & Rössig**
**European Patent Attorneys**
**Cuvilliésstraße 14**
**81679 München (DE)**

(56) References cited:
**WO-A1-98/10285     WO-A2-2013/042111**

• **M. CATTANEO ET AL: "Recommendations for the
standardization of light transmission
aggregometry: a consensus of the working party
from the platelet physiology subcommittee of
SSC/ISTH", JOURNAL OF THROMBOSIS AND
HAEMOSTASIS, vol. 11, no. 6, 1 June 2013
(2013-06-01), pages 1183-1189, XP055484213, GB
ISSN: 1538-7933, DOI: 10.1111/jth.12231**

**Description**

The present invention relates to diagnostic assays. More particularly, the present invention relates to heparin-induced thrombocytopenia assays.

Heparin Induced Thrombocytopenia, also known as HIT, is a prothrombotic and potentially fatal iatrogenic disorder that develops in substantially 5-10 % of patients exposed to heparin (Bell WR, T. P., 1976, Thrombocytopenia occurring during the administration of heparin: A prospective study in 52 patients. Annals of Internal Medicine, 155-160; King DJ, K. J., 1984, Heparin associated thrombocytopenia. Annals of Internal Medicine, 535; Dryjski M, D. H., 1996, Heparin induced thrombocytopenia. European journal of vascular and endovascular surgery, 260-269). HIT involves the development of thrombocytopenia, namely low platelet count, due to the administration of the anticoagulant heparin. HIT predisposes to thrombosis, namely abnormal formation of blood clots inside a blood vessel, because platelets release microparticles that activate thrombin, thereby leading to thrombosis. When thrombosis is identified the condition is termed "heparin-induced thrombocytopenia and thrombosis, also known as HITT. HIT is caused by the formation of antibodies that activate platelets, for example antibodies against complexes of heparin with platelet factor 4 (PF4). Thus, a patient suffering from HIT that receives heparin may develop a new thrombosis, or thrombosis that already exists may worsen in this patient, or the patient's platelet count may fall. Because patients are at high risk of suffering from a thrombotic event, with approximately 30-50 % of patients developing venous and/or arterial thrombosis at the time of HIT diagnosis (Kelton JG, 1986, Heparin-induced Thrombocytopenia. Haemostasis, 173-186), administration of heparin is stopped and patients are speculatively switched onto replacement anticoagulant therapy before diagnosis is confirmed.

Type II HIT, the most serious form of HIT, is mediated by circulating Immunoglobulin G(IgG) antibodies that target complexes of PF4 and Heparin (H) at pharmacological concentration. The IgG: PF4:H complex binds and activates platelets via the $FC_\gamma RII$ receptor leading to thrombin generation and platelet aggregation (Visentin GP, F. S., 1994, Antibodies from Patients with Heparin-induced Thrombocytopenia/Thrombosis Are Specific for Platelet Factor 4 Complexed with Heparin or Bound to Endothelial Cells. Journal of clinical investigation, 81-88). Because thrombotic event is frequent in HIT patients, rapid and reliable diagnosis, allowing immediate switch to alternative anticoagulants, is essential.

Currently recommended HIT diagnostic algorithms for patients whose platelet count drops by over 50% (<50,000/mm$^3$) within 5-14 days of heparin administration incorporate an estimate of clinical probability using a 4Ts score supported by the use of a sensitive immunoassay for initial screening of HIT patients, in order to guide initial management of the HIT positive patients.

An exemplary screening immunoassay for the initial detection of HIT patients is heparin-PF4-ELISA, namely heparin-PF4-enzyme-linked immunosorbent assay. This screening immunoassay is aimed at detecting antibodies against heparin-PF4 complexes. However, heparin-PF4-ELISA detects all circulating antibodies that bind heparin-PF4 complexes and may also falsely detect antibodies that do not cause HIT. Thus, even though the immunoassay is highly sensitive, it lacks specificity. Therefore, those who are found positive in the screening immunoassay, are further tested with a confirmatory more specific functional assay.

Other functional HIT assays test the ability of a serum or plasma sample of a patient to cause platelet aggregation in the presence of heparin. Examples of such assays are: Light Transmission Aggregometry (LTA), Heparin Induced Multi Electrode Aggregometry (HIMEA) and Heparin Induced Platelet Activation (HIPA). In HIPA, for example, serum from the tested patient is mixed with platelets from a donor, in the presence of heparin. Agglutination of the donor's platelets indicates the presence of antibodies against PF4-heparin in the tested patient's serum.

Several functional HIT assays are available, for example the gold standard C-serotonin release assay (SRA). This test uses platelets from several donors and serum from the tested patient. The platelets are loaded with $^{14}$C-serotonin, washed and mixed with serum and heparin. The sample is then tested for the release of serotonin, a marker of platelet activation. If SRA shows high serotonin release, the diagnosis of HIT is confirmed.

Due to their complexity, the aforementioned tests for diagnosing HIT are performed at remote reference laboratories. They are time-consuming because they require batching. In other words, it is impossible to perform these tests for an individual patient on demand. As a result, these prior art tests are performed periodically, for example on a monthly basis. The outcome of this situation is that test results are available in a delay of several weeks. Therefore, the prior art test for diagnosing HIT are inappropriate for emergency situations when a rapid answer is needed for critical care decision making, for example during a cardiac surgery, or during extracorporeal membrane oxygenation (ECMO) procedure. One outcome of this situation is that until results of the tests are obtained, the patient may have been getting a wrong treatment, which may be deleterious, or even lethal. In addition, due to the lack of test results in the time of examination by a physician, suspected HIT patients may receive alternative anticoagulants that are 100-200 times more expensive than heparin, or more difficult to handle than heparin, or associated with bleeding events.

Another drawback of the prior art HIT assays is that they require the usage of donor's platelets to be incubated with a plasma or serum sample of the examined patient. The reason for this is that patient's own platelets cannot be used because of the long period of time between the bleeding of the patient and the test itself.

In addition, the prior art HIT assays are not fully consistent with the physio-pathology of HIT. In HIT, platelet activation is caused by binding of the constant fragment (Fc) of an antiheparin-PF4 complex antibody to a platelet membrane Fc receptor (FcγRIIa) - a low-affinity receptor for the Fc of Immunoglobulin G (IgG), which is also found on neutrophils, monocytes and macrophages. However, there is polymorphism of the Fc receptor, and the receptor structure might even be more important that the antibody concentration for heparin induced platelet activation. This may thus potentially create a diagnosis bias if the donor's platelets have a different morphism than the patient's platelets, resulting in lower accuracy and misdiagnosis accordingly.

Another drawback of the prior art HIT assays relates to the platelets used in the assay. Some of the prior art HIT assays are performed with platelets collected from donors on the basis of the knowledge that these platelets are well activated by anti-heprain-PF4 antibodies. Normally, the platelet donors are laboratory staff members. This raises both an ethical issue and a methodological issue. Regarding the ethical issue, in some countries, for example Belgium, the practice of drawing blood from laboratory personnel to be used in diagnostic assays performed in the laboratory, is forbidden. The methodological issue relates to the fact that laboratory staff members cannot donate platelets repeatedly on a regular basis. In addition, they are not available at any time, for example when an urgent assay is required after the working hours of the laboratory personnel. Another methodological is that in some assays, for example SRA, there is a need to wash the donor's platelets. This adds a step in the assay process and an addition may inadvertently activate the platelets prior to their exposure to the serum or plasma sample of the tested patient.

Furthermore, as a result of the long time that passes until results of the prior art assays, for example SRA and HIPA, are obtained, it is almost impossible to repeat these assays. A person skilled in the art may acknowledge the clinical significance of not being able to repeat an assay when needed.

As mentioned above, due to their complexity and the need to perform these assays in remote reference laboratories, the prior art HIT assays, for example SRA and HIPA, are performed only when an anti-heparin-PF4 antibody screening immunoassay, for example heparin-PF4-ELISA, gives a positive result.

Another drawback of prior art HIT assays is that in some of them, for example HIPA, the results are based on visual inspection of micro titer plates. This requires operation of the assay by an experienced laboratory practitioner. Furthermore, since visual inspection is subjective, accuracy and reproducibility of the prior art assays are questionable.

In addition, the prior art HIT assays are not standardized. For example, SRA is performed according to a different protocol in each laboratory. Other prior art methods, for example impedance aggregometry, for example, Multiplate® Analysis, require that each laboratory performing this assay will define its own clinical cut-off, discriminating between HIT$^+$ and HIT" patients. This limitation translates into increased inter-laboratory variability of assay performance and limited ability to compare results obtained by different laboratories, for example in the context of clinical studies or clinical practice, for example when there is a need to compare results of assays for the same patient that were obtained from different laboratories. Therefore, results from different laboratories cannot be easily compiled without complex statistical meta-analysis.

To summarize, the two major issues in HIT testing are the limited performance of immunoassays in terms of specificity, and the turnaround time as well as the other aforementioned drawbacks of functional assays. These issues raise concerns of overdiagnosing and over-treating HIT, which expose a large number of thrombocytopenic patients to costly alternative anticoagulants and their attendant 10-20 % risk of major bleeding, with clinical and economic impacts likely to be substantial. As it stands, HIT diagnosis is still in need of a fast and standardized test that can be straightforwardly accessible to hemostasis laboratories and with a high specificity.

WO9810285A1 discloses a flow cytometry assay for the determination of heparin-induced thrombocytopenia (HIT) in a patient's serum or plasma sample, the method comprising:

incubating platelets with the patient' s serum or plasma sample in the presence of either a low concentration of heparin (patient low heparin mix) or a high concentration of heparin (patient high heparin mix);
incubating platelets in the presence (positive control mix) or absence (negative control mix) of a platelet activator;
quantifying platelets and activated platelets in the patient low heparin mix, the patient high heparin mix, the positive control mix and the negative control mix.

The invention aims at a reliable and efficient method for assaying a heparin-induced thrombocytopenia.

This technical problem is solved by the method of claim 1. Advantageous embodiments are indicated in further claims.

According to one embodiment, the incubating of the platelets and the quantifying of the platelets and activated platelets are in a one-step incubation, wherein

the patient low heparin mix comprises:

a sample of serum or plasma obtained from a patient;

a sample containing platelets;
a low dose of heparin
a labeled detection element of platelets;
a labeled detection element of activated platelets, and
a diluent;

the patient high heparin mix comprises:

a sample of serum or plasma obtained from a patient;
a sample containing platelets;
a high dose of heparin;
a labeled detection element of platelets,
a labeled detection element of activated platelets, and
a diluent;

the positive control mix comprises:

a sample containing platelets;
platelet activator;
a labeled detection element of platelets;
a labeled detection element of activated platelets, and
a diluent,
and

the negative control mix comprises:

a sample containing platelets;
a labeled detection element of platelets;
a labeled detection element of activated platelets, and
a diluent.

According to another embodiment, the quantifying of the platelets is according to a level of a signal obtained from the labeled detection element of the platelets, and the quantifying of the activated platelets is according to a level of a signal obtained from the labeled detection element of the activated platelets.

According to yet another embodiment, the incubating of the platelets and the quantifying of the platelets and activated platelets are in a two-step incubation, comprising a first incubation and a second incubation,
wherein the first incubation is of:

a patient low heparin first mix comprising:

a sample of serum or plasma obtained from a patient;
a sample containing platelets, and
a low dose of heparin;

a patient high heparin first mix comprising:

a sample of serum or plasma obtained from a patient;
a sample containing platelets, and
a high dose of heparin;

a positive control first mix comprising:

sample containing platelets;
a platelet activator, and
a diluent;
and

a negative control first mix comprising:

sample containing platelets, and
a diluent,

and the second incubation is of:

a patient low heparin second mix comprising:

an aliquot of the patient low heparin first mix after incubation;
a labeled detection element of platelets, and
a labeled detection element of activated platelets;

a patient high heparin second mix comprising:

an aliquot of the patient high heparin first mix after incubation;
a labeled detection element of platelets, and
a labeled detection element of activated platelets;

a positive control second mix comprising:

an aliquot of the positive control first mix;
a labeled detection element of platelets, and
a labeled detection element of activated platelets.

According to still another embodiment, instead of adding the sample containing platelets to the patient low heparin first mix and the patient high heparin first mix, the sample containing platelets is added to the patient low heparin second mix and the patient high heparin second mix.

According to a further embodiment, the calculating cut-off values comprises:

Measuring and calculating HEPLA indices of serum or plasma samples from donors not suffering from HIT;
calculating a mean value and standard deviation (SD) value of the HEPLA index values of the samples obtained from donors not suffering from HIT, and
calculating the cut-off value by multiplying the SD value three times (3SD) and two times (2SD) and adding the obtained product to the mean value, while mean value plus 3SD (3SD cut-off) includes 99% of donors not suffering from HIT and mean value plus 2SD (2SD cut-off) includes 95% of donors not suffering from HIT.

According to yet a further embodiment, the determining whether a patient suffers of HIT or not by comparing the HEPLA index of the patient with the cut-off values comprises the following decisions:

if the HEPLA index of the patient is higher than the 3SD cut-off, the patient suffers from HIT;
if the HEPLA index of the patient is between the 2SD cut-off and the 3SD cut-off, the patient may or may not suffer from HIT and it is optionally recommended to repeat the assay with a fresh sample containing platelets, and
if the HEPLA index of the patient is lower than the 2SD cut-off, the patient does not suffer from HIT.

## DESCRIPTION OF THE PREFERRED EMBODIMENTS

Before explaining at least one embodiment in detail, it is to be understood that the subject matter is not limited in its application to the details of construction and the arrangement of the components set forth in the following description. The subject matter is capable of other embodiments or of being practiced or carried out in various ways. Also, it is to be understood that the phraseology and terminology employed herein is for the purpose of description and should not be regarded as limiting.

For clarity, non-essential elements were omitted from some of the drawings.

In contrast to prior art HIT assays, the HIT assay of the present subject matter can be performed on demand, even for one sample, whereas for the prior art functional HIT assays, like SRA and HIPA, are performed in batches. In addition, the HIT assay of the present subject matter provides results in a short time and can be performed in emergency setting. In contrast to the prior art functional HIT assays, the HIT assay of the present subject matter may be performed with healthy platelets from any type of source that are available, for example in blood banks, including even platelets of the tested patient. These platelets can be isolated in a PRP suspension or in place used directly from whole blood. Furthermore, the HIT assay of the present subject matter may comprise only one step or two, and it may reuse previously established settings of the measuring device. This feature renders the HIT assay of the present subject user friendly as it does not require the involvement of a specialized cytometry practitioner. Due to its aforementioned features, specifically the simplicity of the method, the short time it takes to get results and the high availability of platelets, the HIT assay of the present subject matter is easily repeatable, namely the assay can be performed again immediately when needed. This is important in cases when there is a need to know immediately whether the patient has anti-heparin-PF4 antibodies that may activate platelets. In contrast to prior art HIT assays, the HIT assay of the present subject matter can provide such an answer easily and rapidly. In other words, in contrast to prior art HIT assays, the HIT assay of the present subject matter provides an immediate and fast test for confirming the clinical state of the patient regarding his reaction to administration to heparin. Another advantage of the HIT assay of the present subject matter, compared to prior art functional HIT assays, is that the HIT assay of the present subject matter can be performed either before, or instead, or in parallel to the screening immunoassay, whereas prior art functional HIT assays are performed only after the screening immunoassay, and only for patients who obtained positive results in the screening immunoassay. Thus, the HIT assay of the present subject matter allows determination of the state of HIT reaction in a patient with increased confidence, especially since some incidents of HIT may be caused by antibodies different than those the are detected by the screening immunoassay. In other words, the HIT assay of the present subject matter allows the diagnosis of HIT with a greater certainty than prior art HIT assays. Another benefit of the HIT assay of the present subject matter is its simplicity compared to prior art HIT assays, the ability to perform the assay on a compact automated benchtop flow cytometer, without a requirement for a specific laboratory infrastructure, and with the need for a well experienced laboratory practitioner for performing the assay. This benefit broadens the availability of HIT assays to patients to a large extent, compared to prior art HIT assays. Still another benefit of the HIT assay of the present subject matter is that in contrast to prior art HIT assays it is standardized. This allows comparison of results obtained in different setting, whereas in prior art HIT assays it is impossible. This allows compilation, for example digital compilation of results obtained in different laboratories into a centralized database, thus enabling large multicenter observational studies, or providing a benchmark for laboratory practice. Not to mention the medical and economical benefits of this ability.

The present subject matter provides a HIT assay. The present subject matter also provides a method for diagnosing HIT. The assay and/or method may comprise:

incubating platelets with a patient's serum or plasma sample in the presence of either a low concentration of heparin or a high concentration of heparin;
incubating platelets in the presence (positive control) or absence (negative control) of a platelet activator;
quantifying platelets and activated platelets in the aforementioned four incubations;
calculating percentage of activated platelets within the platelets for each of the aforementioned four incubations and obtaining the following values:

percentage of activated platelets in serum or plasma incubated with a low concentration of heparin [%R(Low)];
percentage of activated platelets in serum or plasma incubated with a high concentration of heparin [%R(High)];
percentage of activated platelets in the positive control [%R(Ct+)], and percentage of activated platelets in the negative control [%R(Ct-)];

calculating a heparin platelet activation (HEPLA) index by dividing the difference between %R(Low) and %R(High) by the difference between %R(Ct+) and %R(Ct-) and multiplying the obtained quotient with 100;
measuring and calculating a HEPLA indices of serum or plasma samples from donors not suffering from HIT;
calculating cut-off values from the HEPLA indices of serum or plasma samples from donors not suffering from HIT, and determining whether a patient suffers of HIT or not by comparing the HEPLA index of the patient with the cut-off values.

The assay and/or method may comprise a step of providing a sample of serum or plasma obtained from a patient. According to one embodiment, the sample of serum or plasma obtained from a patient is a serum sample. According to another embodiment, the sample of serum or plasma obtained from a patient is a plasma sample. The sample of serum or plasma may be prepared from a whole blood sample withdrawn from a patient. In order to obtain a plasma sample the whole blood is collected in a test tube containing an anticoagulant that is not heparin, for example citrate. In order to obtain a serum sample, the whole blood is collected in a test tube not containing an anticoagulant. The plasma or serum may be separated from blood cells by centrifugation of the whole blood, for example at substantially 2,000 g, for substantially 10 minutes, at substantially 25°C. The plasma or serum obtained after the centrifugation may be used directly after preparation, namely fresh sample, in the HIT assay. Alternatively, the plasma or serum sample may be stored at substantially-80°C and used on a later stage. Before usage, the frozen plasma or serum samples may be thawed, for example by bringing them to ambient temperature, or substantially 37°C, and the like. According to one embodiment, the plasma or serum sample may be filtered before the HIT test, for example with a 0.2 $\mu$m filter in order to avoid artefactual activation of platelets by contaminants that may be present in the plasma or serum sample. To summarize, the HIT assay comprises: providing a sample of serum or plasma from a patient.

The assay and/or method may also comprise a step of preparing a sample containing platelets. According to one embodiment, the sample containing platelets is a platelet-rich plasma, also known as PRP. According to another embodiment, the sample containing platelets is whole blood.

The preparation of PRP comprises providing a whole blood sample from a healthy donor, namely a donor not suffering from HIT, or a tested patient. The whole blood sample is collected in a test tube containing an anticoagulant that is not heparin, for example citrate. According to one embodiment the volume ratio is 1 volume anticoagulant and 9 volume whole blood sample from a healthy donor. According to another embodiment, before collecting the whole blood sample from the healthy donor, a sample of whole blood, for example substantially 3-4 ml whole blood, is collected from the healthy donor in a separate test tube and discarded. The whole blood is collected from the patient while minimizing shear stress, for example by using a needle in a size of 21G, using a tourniquet and the like. After the whole blood sample is collected it rests at ambient temperature, for example at a temperature range of substantially 20-25°C for at least substantially 30 minutes. Then, the whole blood sample is centrifuged, for example at substantially 200 g, for substantially 5 minutes, at ambient temperature, for example in the range of substantially 20-25°C. According to one embodiment, the centrifugation is without brake. After centrifugation, the supernatant, which is the PRP from a healthy donor, is delicately transferred to a new test tube, for example a polypropylene test tube, and kept at ambient temperature, for example at a temperature range of substantially 20-25°C, in continuous slow agitation, for example 10 rpm. According to one embodiment, a preferable time period between the start of centrifugation and reading the assay results by cytometry is substantially 3 hours. According to another embodiment, a recommended time period for storing platelets in whole blood is substantially 6 hours without shaking. According to a further embodiment, in order to prevent artefactual activation of platelets in the PRP, the PRP is gently treated, for example, the amount of manipulations of the PRP is minimal, the PRP is not mixed by vortex, and before each use the PRP is delicately shaken between the fingers in order to gently resuspend the platelets in the PRP.

The assay and/or method may further comprise either a one-step incubation or a two-step incubation.

One-step incubation

The one step incubation comprises a step of preparing a patient low heparin mix and a patient high heparin mix.
The patient low heparin mix comprises:

a sample of serum or plasma obtained from a patient;
a sample containing platelets;
a low dose of heparin
a labeled detection element of platelets;
a labeled detection element of activated platelets, and
a diluent.

The patient high heparin mix comprises:

a sample of serum or plasma obtained from a patient;
a sample containing platelets
a high dose of heparin;
a labeled detection element of platelets,
a labeled detection element of activated platelets, and
a diluent.

According to one embodiment, the low dose of heparin is heparin at a final concentration ranging from substantially 0.3 to 1 IU/ml, preferably substantially 0.3 IU/ml. According to another embodiment, the high dose of heparin is heparin at a final concentration ranging from substantially 30 to 500 IU/ml, preferably 100 IU/ml. Any type of heparin known in the art is under the scope of the present subject matter, for example standard porcine heparin-sodium, more particularly standard porcine heparin-sodium 5,000 IU/ml (Sanofi, France).

According to one embodiment, the patient low heparin mix and the patient high heparin mix are in a total volume of 50 μl, of which the volume of the sample of serum or plasma obtained from a patient is 10 μl, the volume of sample containing platelets is 10 μl, and the volume of a heparin stock solution, either for the low dose of heparin or the high dose of heparin, is 5 μl.

According to a preferred embodiment, the sample containing platelets is fresh when added to the mixes. According to another embodiment, the sample containing platelets is added to the mix within substantially three hours after the preparation of the sample containing platelets.

The one-step incubation may further comprise a step of incubation, comprising:

incubating the patient low heparin mix and the patient high heparin mix for activating the platelets by specific antibodies present in the sample of serum or plasma from a patient.

According to one embodiment, the incubation is for substantially 30 minutes with gentle shaking in the dark. According to another embodiment, the incubation is at ambient temperature. According to yet another embodiment, the temperature is at the range of substantially 20-25°C.

The one-step incubation may further comprise a step of preparing a positive control mix and a negative control mix.

The positive control mix comprises:

a sample containing platelets;
platelet activator;
a labeled detection element of platelets;
a labeled detection element of activated platelets, and
a diluent.

The negative control mix comprises:

a sample containing platelets;
a labeled detection element of platelets;
a labeled detection element of activated platelets, and
a diluent.

Any platelet activator known in the art is under the scope of the present matter, for example but not limited to thrombin receptor activating peptide (TRAP), a calcium ionophore, arachidonic acid, adenosine diphosphate (ADP), thrombin and the like.

The positive control mix is aimed at obtaining activated platelets by the platelet activator, and the negative control mix is aimed at obtaining non-activated platelets, since the platelet activator is absent in the negative control mix. According to one embodiment, in order to activate the platelets in the positive control mix, the positive control mix is incubated for a period of time suited for activating the platelets with the platelet activator. According to another embodiment, the positive control is incubated for substantially 30 minutes under gentle shaking. According to yet another embodiment, the positive control mix is incubated in the dark. According to a further embodiment, the negative control mix is incubated similarly to the positive control mix.

According to one embodiment, the positive control mix and the negative control mix are in a total volume of substantially 50 $\mu$l, of which the volume of sample containing platelets is substantially 10 $\mu$l. According to another embodiment, the platelet activator in the positive control mix is in a saturation concentration. For example, when the platelet activator is TRAP, the concentration of TRAP is substantially 50 $\mu$M.

The labeled detection element of platelets is any element known in the art that is configured to detect platelets and labeled with a marker that is configured to be used in flow cytometry. The label may be any light emitting molecule known in the art, or any fluorochrome known in the art. The labeled detection element of platelets may be any marker or molecule binding specifically to platelets, preferably a labeled antibody directed against an antigen that is specific to platelets, for example a labeled antibody directed against platelet glycoprotein IIb/IIIa, for example labeled anti-CD41 antibody, labeled anti-CD41a antibody and the like. According to an additional embodiment, the antibody is any type of antibody known in the art. The antibody may be either polyclonal, or preferably monoclonal.

The labeled detection element of activated platelets is any element known in the art that is configured to detect activated platelets and labeled with a marker that is configured to be used in flow cytometry. The label may be any light emitting molecule known in the art, or any fluorochrome known in the art. The labeled detection element of activated platelets may be any marker or molecule binding specifically to activated platelets, preferably a labeled antibody directed against an antigen that specific to activated platelets, for example a labeled antibody directed against activated platelet p-selectin, for example labeled anti-CD62p antibody and the like. According to an additional embodiment, the antibody is any type of antibody known in the art. The antibody may be either polyclonal, or preferably monoclonal.

According to one embodiment, the detection element of platelets and detection element of activated platelets are labeled each with a fluorescent label, or any light emitting molecule. According to another embodiment, the excitation and emission spectra of the fluorescent label of the detection element of platelets are different from the excitation and emission spectra of the fluorescent label of the detection element of activated platelets. This difference in the excitation and emission spectra allows distinct excitation and detection of the fluorescent emissions of the labels simultaneously when present together in a mix.Any combination of labels that allows simultaneous excitation and emission detection of the labels is under the scope of the present subject matter. An exemplary combination is Fluorescein isothiocyanate (FITC) and Phycoerythrin (PE). Thus, one of the detection elements may be labeled with FITC and the other one with PE. Furthermore, as mentioned above, any type of antibodies known in the art is under the scope of the present subject matter. Thus, for example, the mix may comprise a PE conjugated anti-CD41a monoclonal antibody and a FITC conjugated anti-CD62p monoclonal antibody.

According to one embodiment, the patient low heparin activation detection mix, a patient high heparin activation detection mix, a positive control detection mix and a negative control detection mix are in a total volume of substantially 50 μl, of which the volume of the corresponding patient low heparin mix after incubation, patient high heparin mix after incubation, positive control mix and negative control mix is substantially 5μl.

The one-step incubation may further comprise diluting of the incubated patient low heparin activation detection mix, incubated patient high heparin activation detection mix, incubated positive control detection mix and incubated negative control detection mix, with a biological compatible buffer. According to another embodiment, the biological compatible buffer is phosphate-buffer-saline (PBS), as known in the art. This embodiment relates to any diluent mentioned herein. For example, when the volume of the incubated patient low heparin activation detection mix, incubated patient high heparin activation detection mix, incubated positive control detection mix and incubated negative control detection mix is in a volume of substantially 50 μl, the volume of the biological compatible buffer is substantially 450 μl, giving rise to a total volume of 500 μl.

Two-step incubation

The two-step incubation may comprise a step of preparing a patient low heparin first mix and a patient high heparin first mix.

The patient low heparin first mix comprises:

a sample of serum or plasma obtained from a patient;
a sample containing platelets, and
a low dose of heparin.

The patient high heparin first mix comprises:

a sample of serum or plasma obtained from a patient;
a sample containing platelets, and
a high dose of heparin.

Embodiments related to the low dose of heparin and high dose of heparin are similar to the corresponding embodiment described in the one-step incubation.

According to one embodiment, the sample containing platelets may be added to the patient low heparin first mix and the patient high heparin first mix, as described above.

According to another embodiment, the sample containing platelets may be added to the patient low heparin second mix and the patient high heparin second mix.

According to one embodiment, the patient low heparin first mix and the patient high heparin first mix are in a total volume of 50 μl, of which the volume of the sample of serum or plasma obtained from a patient is 10 μl, the volume of sample containing platelets is 10 μl, and the volume of a heparin stock solution, wither for the low dose of heparin or the high dose of heparin, is 5 μl.

The two-step incubation may further comprise a step of a first incubation, comprising:

incubating the patient low heparin first mix and the patient high heparin first mix for activating the platelets by specific antibodies present in the sample of serum or plasma from a patient.
According to one embodiment, the first incubation is for substantially an hour.
According to another embodiment, the incubation is at ambient temperature. According to yet another embodiment, the temperature is at the range of substantially 20-25°C.

The two-step incubation may further comprise a step of preparing a positive control first mix and a negative control first mix.

The positive control first mix comprises:

sample containing platelets;

a platelet activator, and
a diluent.

The negative control first mix comprises:

sample containing platelets, and
a diluent.

Embodiments related to the platelet activator are similar to the corresponding embodiments described in the one-step incubation.

Also, in the two-step incubation the positive control first mix is aimed at obtaining activated platelets by the platelet activator, and the negative control first mix is aimed at obtaining non-activated platelets, since the platelet activator is absent in the negative control first mix.

According to one embodiment, in order to activate the platelets in the positive control first mix, the positive control first mix is incubated for a period of time suited for activating the platelets with the platelet activator. According to another embodiment, the positive control first mix is incubated for substantially 15 minutes. According to yet another embodiment, the positive control first mix is incubated in the dark. According to a further embodiment, the negative control first mix is incubated similarly to the positive control mix.

According to one embodiment, the positive control first mix and the negative control first mix are in a total volume of substantially 50 $\mu$l, of which the volume of sample containing platelets is substantially 10 $\mu$l. According to another embodiment, the platelet activator in the positive control first mix is in a saturation concentration. For example, when the platelet activator is TRAP, the concentration of TRAP is substantially 50 $\mu$M.

The two-step incubation may further comprise a step of preparing a patient low heparin second mix and a patient high heparin second mix, a positive control second mix and a negative control second mix.

The patient low heparin second mix comprises:

an aliquot of the patient low heparin first mix after incubation;
a labeled detection element of platelets, and
a labeled detection element of activated platelets.

The patient high heparin second mix comprises:

an aliquot of the patient high heparin first mix after incubation;
a labeled detection element of platelets, and
a labeled detection element of activated platelets.

The positive control second mix comprises:

an aliquot of the positive control first mix;
a labeled detection element of platelets, and
a labeled detection element of activated platelets.

The negative control second mix comprises:

An aliquot of the negative control first mix;
a labeled detection element of platelets, and
a labeled detection element of activated platelets.

Embodiments related to the labeled detection element of platelets and the labeled detection element of activated platelets are similar to the corresponding embodiments described in the one-step incubation.

According to one embodiment, the patient low heparin second mix, the patient high heparin second mix, the positive control second mix and the negative control second mix are in a total volume of substantially 50 $\mu$l, of which the volume of the corresponding patient low heparin first mix after incubation, the patient high heparin first mix after incubation, the positive control first mix and the negative control first mix is substantially 5$\mu$l.

The two-step incubation may further comprise a step of a second incubation comprising incubating the patient low heparin second mix, the patient high heparin second mix, the positive control second mix and the negative control second mix, in order to allow binding of each one of the labeled detection elements to its target, for example when the labeled detection element is an antibody, the second incubation is aimed at allowing binding of the antibody to its specific antigen. According to one embodiment, the second incubation is for substantially 15 minutes. According to another embodiment, the second incubation is at ambient temperature. According to yet another embodiment, the second incubation is at a temperature range of substantially 20-25°C. According to still another embodiment, the second incubation is under any condition known in the art that does not harm fluorescent labels, for example in the dark.

The assay and/or method may further comprise diluting of the incubated patient low heparin second mix, the incubated patient high heparin second mix, the incubated positive control second mix and the incubated negative control second mix, with a diluent. Embodiments related to the diluent and the dilution of the second mixes of the diluent are similar to the corresponding embodiments described in the one-step incubation.

Calculations and interpretations

The assay and/or method may further comprise, after either the one-step incubation or the two-step incubation a step of calculating the percentage of activated platelets from total platelets in each one of the diluted incubated patient low heparin activation detection mix, designated hereinafter "%R(Low)"; diluted incubated patient high heparin activation detection mix"%R(High)"; diluted incubated positive control detection mix, designated hereinafter "%R(Ct+)", and diluted incubated negative control detection mix, designated hereinafter "%R(Ct-)". The calculations may be performed by the device that reads the samples.

According to one embodiment, the calculating of the percentage of activated platelets from total platelets, the calculating comprising:

determining an amount of total platelets according to the level of a signal obtained from the labeled detection element of platelets;
determining an amount of activated platelets according to the level of a signal obtained from the labeled detection element of activated platelets within the amount of total platelets.

The assay and/or method may further comprise a step of calculating a ratio of heparin activated platelets over potentially activatable platelets, designated hereinafter "HEPLA index". The HEPLA index is calculated by dividing the difference between %R(Low) and %R(High) by the difference between %R(Ct+) and %R(Ct-) and multiplying the obtained quotient with 100. The following formula summarizes the calculation of HEPLA index:

$$\% \text{ HEPLA index} = 100 * [\%R(Low) - \%R(High)] / [\%R(Ct+) - \%R(Ct-)]$$

The assay and/or method may further comprise a step of interpretation of the HEPLA index based on a comparison with calculated cut-off values, obtained by a method for calculating cut-off values, comprising:

measuring and calculating the HEPLA indices of serum or plasma samples from donors not suffering from HIT;
calculating a mean value and standard deviation (SD) value of the HEPLA index values of the samples obtained from donors not suffering from HIT, and
calculating the cut-off value by multiplying the SD value three times (3SD) and two times (2SD) and adding the obtained product to the mean value, while mean value plus 3SD (3SD cut-off) includes 99% of donors not suffering from HIT and mean value plus 2SD (2SD cut-off) includes 95% of donors not suffering from HIT.

According to one embodiment, the 2SD cut-off value of the HEPLA index may be substantially 9.6% and the 3SD cut-off value may be substantially 13%. It should be noted that the cut-off values described above are only exemplary, and that the cut-off values could be different function of the one-step or two-step method used. It should be noted though, that any cut-off value of the HEPLA index that is obtained for the samples obtained from donor not suffering from HIT is under the scope of the present subject matter.

The assay and/or method may further comprise a step of determining whether a patient suffers from HIT or not, comprising:

comparing a HEPLA index of the patient with the cut-off;
if the HEPLA index of the patient is higher than the 3SD cut-off, the patient suffers from HIT;
if the HEPLA index of the patient is between the 2SD cut-off and the 3SD cut-off, the patient may or may not suffer from HIT and it is optionally recommended to repeat the assay with a fresh sample containing platelets;
if the HEPLA index of the patient is lower than the 2SD cut-off, the patient does not suffer from HIT.

According to one embodiment, the reagents used in the assay and/or method are equilibrated at ambient temperature before use. According to another embodiment, the ambient temperature is at the range of substantially 20-25°C.

It is appreciated that certain features of the subject matter, which are, for clarity, described in the context of separate embodiments, may also be provided in combination in a single embodiment. Conversely, various features of the subject matter, which are, for brevity, described in the context of a single embodiment, may also be provided separately or in any suitable sub combination.

Although the subject matter has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

**Claims**

1. A method for assaying a heparin-induced thrombocytopenia (HIT) in a patient's serum or plasma sample, the method comprising:

   incubating platelets with the patient's serum or plasma sample in the presence of either a low concentration of heparin (patient low heparin mix) or a high concentration of heparin (patient high heparin mix);
   incubating platelets in the presence (positive control mix) or absence (negative control mix) of a platelet activator;
   quantifying platelets and activated platelets in the patient low heparin mix, the patient high heparin mix, the positive control mix and the negative control mix;
   calculating percentage of activated platelets within the platelets for each of the aforementioned mixes and obtaining the following values:

   percentage of activated platelets in the patient low heparin mix [%R(Low)];
   percentage of activated platelets in the patient high heparin mix [%R(High)];
   percentage of activated platelets in the positive control mix [%R(Ct+)], and
   percentage of activated platelets in the negative control mix [%R(Ct-)];

   calculating a heparin platelet activation (HEPLA) index by dividing the difference between %R(Low) and %R(High) by the difference between %R(Ct+) and %R(Ct-) and multiplying the obtained quotient with 100;
   measuring and calculating HEPLA indices of serum or plasma samples from donors not suffering from HIT;
   calculating cut-off values from the HEPLA indices of serum or plasma samples from donors not suffering from HIT, and
   determining whether a patient suffers of HIT or not by comparing the HEPLA index of the patient with the cut-off values.

2. The method of claim 1, wherein the incubating of the platelets and the quantifying of the platelets and activated platelets are in a one-step incubation, wherein

   the patient low heparin mix comprises:

   a sample of serum or plasma obtained from a patient;
   a sample containing platelets;
   a low dose of heparin
   a labeled detection element of platelets;
   a labeled detection element of activated platelets, and
   a diluent;

   the patient high heparin mix comprises:

   a sample of serum or plasma obtained from a patient;
   a sample containing platelets
   a high dose of heparin;
   a labeled detection element of platelets,
   a labeled detection element of activated platelets, and
   a diluent;

the positive control mix comprises:

a sample containing platelets;
platelet activator;
a labeled detection element of platelets;
a labeled detection element of activated platelets, and
a diluent,
and

the negative control mix comprises:

a sample containing platelets;
a labeled detection element of platelets;
a labeled detection element of activated platelets, and
a diluent.

3. The method of claim 2, wherein the quantifying of the platelets is according to a level of a signal obtained from the labeled detection element of the platelets, and the quantifying of the activated platelets is according to a level of a signal obtained from the labeled detection element of the activated platelets.

4. The method of claim 1, wherein the incubating of the platelets and the quantifying of the platelets and activated platelets are in a two-step incubation, comprising a first incubation and a second incubation,

wherein the first incubation is of:

a patient low heparin first mix comprising:

a sample of serum or plasma obtained from a patient;
a sample containing platelets, and
a low dose of heparin;

a patient high heparin first mix comprising:

a sample of serum or plasma obtained from a patient;
a sample containing platelets, and
a high dose of heparin;

a positive control first mix comprising:

sample containing platelets;
a platelet activator, and
a diluent;
and

a negative control first mix comprising:

sample containing platelets, and
a diluent,

and the second incubation is of:

a patient low heparin second mix comprising:

an aliquot of the patient low heparin first mix after incubation;
a labeled detection element of platelets, and
a labeled detection element of activated platelets;

a patient high heparin second mix comprising:

an aliquot of the patient high heparin first mix after incubation;
a labeled detection element of platelets, and
a labeled detection element of activated platelets;

a positive control second mix comprising:

an aliquot of the positive control first mix;
a labeled detection element of platelets, and
a labeled detection element of activated platelets.

5. The method of claim 4, wherein the quantifying of the platelets is according to a level of a signal obtained from the labeled detection element of the platelets, and the quantifying of the activated platelets is according to a level of a signal obtained from the labeled detection element of the activated platelets.

6. The method of claim 4, wherein instead of adding the sample containing platelets to the patient low heparin first mix and the patient high heparin first mix, the sample containing platelets is added to the patient low heparin second mix and the patient high heparin second mix.

7. The method of claim 1, wherein the calculating cut-off values comprises:

Measuring and calculating HEPLA indices of serum or plasma samples from donors not suffering from HIT;
calculating a mean value and standard deviation (SD) value of the HEPLA index values of the samples obtained from donors not suffering from HIT, and
calculating the cut-off value by multiplying the SD value three times (3SD) and two times (2SD) and adding the obtained product to the mean value, while mean value plus 3SD (3SD cut-off) includes 99% of donors not suffering from HIT and mean value plus 2SD (2SD cut-off) includes 95% of donors not suffering from HIT.

8. The method of claim 7, wherein the determining whether a patient suffers of HIT or not by comparing the HEPLA index of the patient with the cut-off values comprises the following decisions:

if the HEPLA index of the patient is higher than the 3SD cut-off, the patient suffers from HIT;
if the HEPLA index of the patient is between the 2SD cut-off and the 3SD cut-off, the patient may or may not suffer from HIT and it is optionally recommended to repeat the assay with a fresh sample containing platelets, and
if the HEPLA index of the patient is lower than the 2SD cut-off, the patient does not suffer from HIT.

**Patentansprüche**

1. Verfahren zum Testen einer Heparin-induzierten Thrombozytopenie (HIT) in einer Serum- oder Plasmaprobe eines Patienten, wobei das Verfahren umfasst:

Inkubieren von Thrombozyten mit der Serum- oder Plasmaprobe des Patienten in Gegenwart von entweder einer niedrigen Heparinkonzentration (Patienten-Mischung mit niedriger Heparinkonzentration) oder einer hohen Heparinkonzentration (Patienten-Mischung mit hoher Heparinkonzentration);
Inkubieren von Thrombozyten in Gegenwart (positive Kontrollmischung) oder Abwesenheit (negative Kontrollmischung) eines Thrombozytenaktivators;
Quantifizieren der Thrombozyten und der aktivierten Thrombozyten in der Patienten-Mischung mit niedriger Heparinkonzentration, der Patienten-Mischung mit hoher Heparinkonzentration, der positiven Kontrollmischung und der negativen Kontrollmischung; Berechnen des Prozentsatzes der aktivierten Thrombozyten innerhalb der Thrombozyten für jede der vorgenannten Mischungen und Ermitteln der folgenden Werte:

Prozentsatz der aktivierten Thrombozyten in der Patienten-Mischung mit niedriger Heparinkonzentration [%R(Low)];
Prozentsatz der aktivierten Thrombozyten in der Patienten-Mischung mit hoher Heparinkonzentration [%R(High)];
Prozentsatz der aktivierten Thrombozyten in der positiven Kontrollmischung [%R(Ct+)] und
Prozentsatz der aktivierten Thrombozyten in der negativen Kontrollmischung [%R(Ct-)];

Berechnen eines Heparin-Thrombozytenaktivierungsindex (Heparin Platelet Activation, HEPLA) durch Dividieren der Differenz zwischen %R(Low) und
%R(High) durch die Differenz zwischen %R(Ct+) und %R(Ct-) und Multiplizieren des erhaltenen Quotienten mit 100;
Messen und Berechnen der HEPLA-Indizes von Serum- oder Plasmaproben von Spendern, die nicht an HIT leiden;
Berechnen von Cut-off-Werten aus den HEPLA-Indizes von Serum- oder Plasmaproben von Spendern, die nicht an HIT leiden, und
Bestimmen, ob ein Patient an HIT leidet oder nicht, indem der HEPLA-Index des Patienten mit den Cut-off-Werten verglichen wird.

2. Verfahren nach Anspruch 1, wobei das Inkubieren der Thrombozyten und das Quantifizieren der Thrombozyten und aktivierten Thrombozyten in einer einstufigen Inkubation erfolgen, wobei

    die Patienten-Mischung mit niedriger Heparinkonzentration umfasst:

        eine von einem Patienten entnommene Serum- oder Plasmaprobe;
        eine Probe, die Thrombozyten enthält;
        eine niedrige Heparindosis
        ein markiertes Erkennungselement für Thrombozyten;
        ein markiertes Erkennungselement für aktivierte Thrombozyten, und
        ein Verdünnungsmittel;

    die Patienten-Mischung mit hoher Heparinkonzentration umfasst:

        eine von einem Patienten entnommene Serum- oder Plasmaprobe;
        eine Probe, die Thrombozyten enthält
        eine hohe Heparindosis;
        ein markiertes Erkennungselement für Thrombozyten,
        ein markiertes Erkennungselement für aktivierte Thrombozyten, und
        ein Verdünnungsmittel;

    die positive Kontrollmischung umfasst:

        eine Probe, die Thrombozyten enthält;
        einen Thrombozyten-Aktivator;
        ein markiertes Erkennungselement für Thrombozyten;
        ein markiertes Erkennungselement für aktivierte Thrombozyten, und
        ein Verdünnungsmittel,

    und die negative Kontrollmischung umfasst:

        eine Probe, die Thrombozyten enthält;
        ein markiertes Erkennungselement für Thrombozyten;
        ein markiertes Erkennungselement für aktivierte Thrombozyten, und
        ein Verdünnungsmittel.

3. Verfahren nach Anspruch 2, wobei das Quantifizieren der Thrombozyten gemäß einem Pegel eines Signals erfolgt, das von dem markierten Erkennungselement der Thrombozyten erhalten wird, und das Quantifizieren der aktivierten Thrombozyten gemäß einem Pegel eines Signals erfolgt, das von dem markierten Erkennungselement der aktivierten Thrombozyten erhalten wird.

4. Verfahren nach Anspruch 1, wobei das Inkubieren der Thrombozyten und das Quantifizieren der Thrombozyten und aktivierten Thrombozyten in einer zweistufigen Inkubation erfolgen, die eine erste Inkubation und eine zweite Inkubation umfasst,

    wobei die erste Inkubation eine der folgenden ist:

eine erste Patienten-Mischung mit niedriger Heparinkonzentration, umfassend:

eine von einem Patienten entnommene Serum- oder Plasmaprobe;
eine Probe, die Thrombozyten enthält, und
eine niedrige Heparindosis;

eine erste Patienten-Mischung mit hoher Heparinkonzentration, umfassend:

eine von einem Patienten entnommene Serum- oder Plasmaprobe;
eine Probe, die Thrombozyten enthält, und
eine hohe Heparindosis;

eine erste positive Kontrollmischung, umfassend:

eine Probe, die Thrombozyten enthält;
einen Thrombozytenaktivator, und ein Verdünnungsmittel;

und eine erste negative Kontrollmischung, umfassend:

eine Probe, die Thrombozyten enthält, und
ein Verdünnungsmittel,

und die zweite Inkubation eine der folgenden ist:

eine zweite Patienten-Mischung mit niedriger Heparinkonzentration, umfassend:

ein Aliquot der ersten Patienten-Mischung mit niedriger Heparinkonzentration nach der Inkubation;
ein markiertes Erkennungselement für Thrombozyten, und
ein markiertes Erkennungselement für aktivierte Thrombozyten;

eine zweite Patienten-Mischung mit hoher Heparinkonzentration, umfassend:

ein Aliquot der ersten Patienten-Mischung mit hoher Heparinkonzentration nach der Inkubation;
ein markiertes Erkennungselement für Thrombozyten, und
ein markiertes Erkennungselement für aktivierte Thrombozyten;

eine zweite positive Kontrollmischung, umfassend:

ein Aliquot der ersten positiven Kontrollmischung;
ein markiertes Erkennungselement für Thrombozyten, und
ein markiertes Erkennungselement für aktivierte Thrombozyten.

5. Verfahren nach Anspruch 4, wobei das Quantifizieren der Thrombozyten gemäß einem Pegel eines Signals erfolgt, das von dem markierten Erkennungselement der Thrombozyten erhalten wird, und das Quantifizieren der aktivierten Thrombozyten gemäß einem Pegel eines Signals erfolgt, das von dem markierten Erkennungselement der aktivierten Thrombozyten erhalten wird.

6. Verfahren nach Anspruch 4, wobei anstelle der Zugabe der Thrombozyten enthaltenden Probe zu der ersten Patienten-Mischung mit niedriger Heparinkonzentration und der ersten Patienten-Mischung mit hoher Heparinkonzentration die Thrombozyten enthaltende Probe zu der zweiten Patienten-Mischung mit niedriger Heparinkonzentration und der zweiten Patienten-Mischung mit hoher Heparinkonzentration zugegeben wird.

7. Verfahren nach Anspruch 1, wobei das Berechnen von Cut-off-Werten umfasst:

Messen und Berechnen der HEPLA-Indizes von Serum- oder Plasmaproben von Spendern, die nicht an HIT leiden;
Berechnen eines Mittelwerts und der Standardabweichung (SD) der HEPLA-Indexwerte der Proben von Spendern, die nicht an HIT leiden, und

Berechnen des Cut-off-Werts durch Multiplizieren des SD-Werts mit dem Dreifachen (3SD) und dem Zweifachen (2SD) und Addieren des erhaltenen Produkts zum Mittelwert, wobei der Mittelwert plus 3SD (3 SD-Cut-off) 99 % der Spender einschließt, die nicht an HIT leiden, und der Mittelwert plus 2SD (2SD-Cut-off) 95 % der Spender einschließt, die nicht an HIT leiden.

**8.** Verfahren nach Anspruch 7, wobei das Bestimmen, ob ein Patient an HIT leidet oder nicht, indem der HEPLA-Index des Patienten mit den Cut-off-Werten verglichen wird, die folgenden Entscheidungen umfasst:

ist der HEPLA-Index des Patienten höher als der 3 SD-Cut-off, leidet der Patient an HIT;
liegt der HEPLA-Index des Patienten zwischen dem 2SD-Cut-Off und dem 3SD-Cut-Off, kann der Patient an HIT leiden oder auch nicht, und es wird empfohlen, das Assay mit einer frischen Probe zu wiederholen, die Thrombozyten enthält, und ist der HEPLA-Index des Patienten niedriger als der 2SD-Cut-off, leidet der Patient nicht an HIT.

## Revendications

**1.** Procédé d'analyse d'une thrombocytopénie induite par l'héparine (TIH) dans un échantillon de sérum ou de plasma d'un patient, le procédé comprenant :

l'incubation de plaquettes avec l'échantillon de sérum ou de plasma du patient en présence soit d'une faible concentration d'héparine (faible mix d'héparine patient), soit d'une concentration élevée d'héparine (mix élevé d'héparine patient) ;
l'incubation de plaquettes en présence (mix de contrôle positif) ou en absence (mix de contrôle négatif) d'un activateur de plaquettes ;
la quantification des plaquettes et des plaquettes activées dans le faible mix d'héparine patient, le mix élevé d'héparine patient, le mix de contrôle positif et le mix de contrôle négatif ;
le calcul du pourcentage de plaquettes activées à l'intérieur des plaquettes pour chacun des mix sus-mentionnés et l'obtention des valeurs suivantes :

pourcentage de plaquettes activées dans le faible mix d'héparine patient [%R(Low)] ;
pourcentage de plaquettes activées dans le mix élevé d'héparine patient [%R(High)] ;
pourcentage de plaquettes activées dans le mix de contrôle positif [%R(Ct+)] et
pourcentage de plaquettes activées dans le mix de contrôle négatif [%R(Ct-)] ;

le calcul de l'indice d'activation plaquettaire induite par l'héparine en divisant la différence entre %R(Low) et %R(High) par la différence entre %R(Ct+) et %R(Ct-) et la multiplication du quotient obtenu par 100 ;
la mesure et le calcul des indices d'activation plaquettaire induite par l'héparine des échantillons de sérum ou de plasma de donneurs qui ne souffrent pas de TIH ;
le calcul de valeurs limites d'indices d'activation plaquettaire induite par l'héparine d'échantillons de sérum ou de plasma de donneurs qui ne souffrent pas de TIH et
la détermination du fait de savoir si un patient souffre de TIH ou non en comparant l'indice d'activation plaquettaire induite par l'héparine du patient avec les valeurs limites.

**2.** Procédé selon la revendication 1, l'incubation de plaquettes et la quantification des plaquettes et des plaquettes activées étant dans une incubation en une étape, cependant que

le faible mix d'héparine patient comprend :

un échantillon de sérum ou de plasma obtenu d'un patient ;
un échantillon contenant des plaquettes ;
une faible dose d'héparine ;
un élément de détection marqué de plaquettes ;
un élément de détection marqué de plaquettes activées et
un diluant ;

le mix élevé d'héparine patient comprend :

un échantillon de sérum ou de plasma obtenu d'un patient ;
un échantillon contenant des plaquettes ;
une dose élevée d'héparine ;
un élément de détection marqué de plaquettes ;
un élément de détection marqué de plaquettes activées et
un diluant ;

le mix de contrôle positif comprend :

un échantillon contenant des plaquettes ;
un activateur de plaquettes ;
un élément de détection marqué de plaquettes ;
un élément de détection marqué de plaquettes activées et
un diluant

et

le mix de contrôle négatif comprend :

un échantillon contenant des plaquettes ;
un élément de détection marqué de plaquettes ;
un élément de détection marqué de plaquettes activées et
un diluant.

3. Procédé selon la revendication 2, la quantification des plaquettes étant selon un niveau de signal obtenu par l'élément de détection marqué des plaquettes et la quantification des plaquettes activées étant selon un niveau de signal obtenu par l'élément de détection marqué des plaquettes activées.

4. Procédé selon la revendication 1, l'incubation des plaquettes et la quantification des plaquettes et des plaquettes activées étant dans une incubation à deux étapes comprenant une première incubation et une seconde incubation,

la première incubation étant :

d'un premier faible mix d'héparine patient comprenant :

un échantillon de sérum ou de plasma obtenu d'un patient ;
un échantillon contenant des plaquettes et
une faible dose d'héparine ;

d'un premier mix élevé d'héparine patient comprenant :

un échantillon de sérum ou de plasma obtenu d'un patient ;
un échantillon contenant des plaquettes et
une dose élevée d'héparine ;

d'un premier mix de contrôle positif comprenant :

un échantillon contenant des plaquettes ;
un activateur de plaquettes et
un diluant

et d'un premier mix de contrôle négatif comprend :

un échantillon contenant des plaquettes et
un diluant,

et la seconde incubation étant :

d'un second faible mix d'héparine patient comprenant :

un aliquote du premier faible mix d'héparine patient après incubation ;
un élément de détection marqué de plaquettes et
un élément de détection marqué de plaquettes activées ;

d'un second mix élevé d'héparine patient comprenant :

un aliquote du premier mix élevé d'héparine patient après incubation ;
un élément de détection marqué de plaquettes et
un élément de détection marqué de plaquettes activées ;

d'un second mix de contrôle positif comprenant :

un aliquote du premier mix de contrôle positif ;
un élément de détection marqué de plaquettes et
un élément de détection marqué de plaquettes activées.

5. Procédé selon la revendication 4, la quantification des plaquettes étant selon un niveau de signal obtenu par l'élément de détection marqué des plaquettes et la quantification des plaquettes activées étant selon un niveau de signal obtenu par l'élément de détection marqué des plaquettes activées.

6. Procédé selon la revendication 4, cependant qu'au lieu d'ajouter l'échantillon contenant des plaquettes au premier faible mix d'héparine patient et au premier mix élevé d'héparine patient, l'échantillon contenant des plaquettes est ajouté au second faible mix d'héparine patient et au second mix élevé d'héparine patient.

7. Procédé selon la revendication 1, le calcul des valeurs limites comprenant :

la mesure et le calcul d'indices d'activation plaquettaire induite par l'héparine d'échantillons de sérum ou de plasma de donneurs qui ne souffrent pas de TIH ;
le calcul d'une valeur moyenne et d'un écart-type (SD) des indices d'activation plaquettaire induite par l'héparine d'échantillons obtenus de donneurs qui ne souffrent pas de TIH et
le calcul de la valeur limite en multipliant l'écart-type SD trois fois (3SD) et deux fois (2SD) et l'ajout du produit obtenu à la valeur moyenne, tandis que la valeur moyenne plus 3SD (valeur limite 3SD) comprend 99% des donneurs qui ne souffrent pas de TIH et la valeur moyenne plus 2SD (valeur limite 2SD) comprend 95% des donneurs qui ne souffrent pas de TIH.

8. Procédé selon la revendication 7, le fait de déterminer si un patient souffre de TIH ou non en comparant l'indice d'activation plaquettaire induite par l'héparine du patient avec les valeurs limites comprend les décisions suivantes :

si l'indice d'activation plaquettaire induite par l'héparine du patient est supérieur à la valeur limite 3SD, le patient souffre de TIH ;
si l'indice d'activation plaquettaire induite par l'héparine du patient est entre la valeur limite 2SD et la valeur limite 3SD, le patient peut souffrir de TIH ou non et il est recommandé facultativement de répéter l'analyse avec un échantillon frais contenant des plaquettes et
si l'indice d'activation plaquettaire induite par l'héparine du patient est inférieur à la valeur limite 2SD, le patient ne souffre pas de TIH.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9810285 A1

**Non-patent literature cited in the description**

- **BELL WR, T. P.** Thrombocytopenia occurring during the administration of heparin: A prospective study in 52 patients. *Annals of Internal Medicine,* 1976, 155-160
- **KING DJ, K. J.** Heparin associated thrombocytopenia. *Annals of Internal Medicine,* 1984, 535
- **DRYJSKI M, D. H.** Heparin induced thrombocytopenia. *European journal of vascular and endovascular surgery,* 1996, 260-269
- **KELTON JG.** Heparin-induced Thrombocytopenia. *Haemostasis,* 1986, 173-186
- **VISENTIN GP, F. S.** Antibodies from Patients with Heparin-induced Thrombocytopenia/Thrombosis Are Specific for Platelet Factor 4 Complexed with Heparin or Bound to Endothelial Cells. *Journal of clinical investigation,* 1994, 81-88